# EUROPEAN PATENT SPECIFICATION

(11) **EP 3 090 772 B1**
(45) Date of publication and mention of the grant of the patent: **20.03.2019**
(21) Application number: 14876500.1
(22) Date of filing: 11.04.2014
(51) Int. Cl.: A61M 5/50, A61M 5/32, A61M 5/00

(54) **SYRINGE NEEDLE ASSEMBLY, AND SAFETY SYRINGE AND SAFETY SYRINGE APPARATUS USING SAME**
SPRITZENNADELANORDNUNG UND SICHERHEITSSPRITZE SOWIE SICHERHEITSSPRITZENVORRICHTUNG DAMIT
ENSEMBLE AIGUILLE DE SERINGUE, ET SERINGUE DE SÉCURITÉ ET APPAREIL DE SERINGUE DE SÉCURITÉ L'UTILISANT

(30) Priority: 31.12.2013 KR 20130168218
(43) Date of publication of application: 09.11.2016
(73) Proprietor: Saferwith, Inc., Suwon-si, Gyeonggi-do 443-270 (KR)
(72) Inventor: JANG, Ji Hoon, Seoul 140-050 (KR); PARK, Kyung Taek, Osan-si Gyeonggi-do 447-782 (KR)
(74) Representative: Grauel, Andreas
(86) International application number: PCT/KR2014/003159
(87) International publication number: WO 2015/102162

(56) References cited:
- EP-A1- 2 574 357
- CN-A- 103 357 090
- KR-A- 20050 004 776
- KR-B1- 100 800 426
- KR-B1- 100 917 795
- US-A- 5 242 418
- US-A- 5 472 430
- US-A- 5 478 328
- US-B1- 6 261 264
- US-B1- 6 409 706

## Description

### Technical Field

The present invention relates to a syringe needle assembly, and a safety syringe and a safety syringe device using the syringe needle assembly, and more particularly, to a syringe needle assembly configured to prevent a cross infection by a syringe needle contaminated with a patient's blood after injection, and a safety syringe and a safety syringe device using the syringe needle assembly.

### Background Art

Hospitals are regarded as harmful environments where work-related injuries increase. Hospital workers may be exposed to biological, chemical and physical factors which may cause them to suffer from occupational diseases.

Generally and most commonly, hospital workers may be infected with diseases when a patient's contaminated blood comes into contact with their mucous membrane or a scratch or they are pricked by a syringe needle or various sharp instruments used on patients. Due to so called needlestick injuries, the hospital workers are, due to their profession, in potential danger of being infected with very serious and fatal diseases.

Hospital workers may suffer from injuries by a syringe needle even though the injuries may or may not cause bleeding. For example, the skin of the hospital workers may be torn, scratched or pricked by a disposable syringe or a needle for blood sugar measurement before or after it is contaminated with a patient's blood, or an exposed syringe needle that has been used on a patient and thus contaminated, such as a syringe needle for use in a surgical or invasive measure. Such an injury frequently occurs even in the process of recapping a syringe needle with a needle cap provided for redundant safety.

Blood-borne infections refer to diseases transmitted through blood by germs, such as hepatitis B and C viruses, Human Immunodeficiency Virus (HIV), and treponema pallidum. As measures for using syringe needles are developed and inspection techniques directly handling bloods increase, hospital workers are gradually more likely to be infected with blood-borne infections. There are more than 20 types of diseases transmitted by exposure to blood including virus, bacteria and parasite.

It is reported that many hospital workers are exposed to blood-borne infections and among others, injuries by syringe needles frequently occur.

A safety syringe refers to a syringe to prevent accidental needle stick injury and to a non-reusable syringe configured to remove danger of syringe needle. Conventional safety syringes include a type configured to cover a syringe needle with a cover, a type configured to retract a syringe needle into the syringe, a type configured to perform injection without a needle, etc.

In connection with the type configured to retract the syringe needle, a safety syringe is disclosed in Korean Patent Laid-Open Publication No. 2003-0029744. The safety syringe disclosed in Patent Laid-Open Publication No. 2003-0029744 includes: a cylinder having a neck portion formed on the upper side thereof and a space portion formed therein to be capable of storing an injection liquid therein, a plunger inserted into the cylinder to be movable, an adaptor coupled to the neck portion of the cylinder, and a syringe needle connected to the adaptor. A recess is formed on the adaptor, in which the recess has a cross-sectional area which gradually decreases in size towards the upper end and the central axis of the recess is positioned on the same line as the central axis of the cylinder. A protrusion is formed on the tip end of the plunger, in which the protrusion has a cross-sectional area which gradually decreases in size towards the upper end and is biased from the central axis of the cylinder so that the protrusion may be inserted into the recess formed in the adaptor in a state where at least a part of the protrusion is compressed. The adaptor is rotationally coupled to the neck portion to be removable.

In addition, the type configured to cover the syringe needle with a cover, a protection cap of a disposable safety syringe is disclosed in Korean Patent No. 10-1164447. In the protection cover coupled to the upper portion of the disposable syringe to cover the syringe needle as disclosed in Korean Patent No. 10-1164447, an insertion slot cut in the longitudinal direction is formed on the protection cap so that the upper portion of the syringe including the syringe needle can be inserted into the protection cap laterally, and a fixing groove with a predetermined width in the longitudinal direction is formed by partially cutting a part of the lower end portion of the insertion slot in the circumferential direction. When the syringe is used, the protection cap is moved up in the vertical direction to be separated from the syringe, and after the syringe is used, the upper portion of the syringe is inserted laterally into the protection cap through the insertion slot so that the syringe needle is covered with the protection cover and the protrusion formed around the upper portion of the syringe is inserted into the fixing groove formed on the lower end of the protection cap to be fixed. The longitudinal width of the fixing groove of the protection cap is formed to be minutely different from the thickness of the protrusion of the syringe so that the protection cap is press-fitted to the upper portion of the syringe when the protrusion is inserted into the fixing groove.

Meanwhile, in connection with the type configured to perform injection without a needle, a needle-free syringe is disclosed in Korean Patent No. 10-1052669. In the syringe disclosed in Korean Patent No. 10-1052669, when a press lever is pulled to rotate a latch clockwise, a drum is rotated together with the latch to pull a piston so that a predetermined amount of a medicinal liquid in a medicinal liquid barrel is sucked into a cylinder. When the suction of the medicinal liquid into the cylinder is completed, the engagement state of the latch and the drum is released by pulling a trigger. Then, the piston is moved forward by a restoring force of a spring so that the medicinal liquid within the cylinder can be injected.

However, the conventional safety syringes disclosed in Korean Patent Laid-Open Publication No. 2003-0029744, Korean Patent No. 10-1164447, and Korean Patent No. 10-1052669 employ safety structures which are complicated compared to existing syringes generally used to prevent external exposure of a syringe needle after injection and differentiated from the existing structures. Accordingly, the existing syringes or syringe needles cannot be used as the above-described conventional safety syringes by upgrading the existing syringes or syringe needles, and even new molds should be manufactured.

The conventional safety syringes disclosed in Korean Patent Laid-Open Publication No. 2003-0029744, Korean Patent No. 10-1164447, and Korean Patent No. 10-1052669 have not yet been practically distributed in the medical field mainly due to the costs required for providing safety syringes or the like and financial problems of securing funds according to target patients who should use safety syringes and a use range thereof.

That is, all the interested parties, such as hospitals and legislatures, have agreed to the notion that medical personnel are in danger of cross infections by syringes and the necessity of safety syringes. However, since the manufacturing costs of the safety syringes are several times or dozens of times higher than those of ordinary syringes due to the structural complexity as described above, agreement in the legislature, the burden of finance, social recognition, etc. has not yet been settled.

Accordingly, what is needed is a safety syringe which is configured to be applicable to an existing syringe or syringe needle when used by upgrading the existing syringe or syringe needle, has a simple and safe structure, and is configured to be used easily and conveniently.
Documents US 5 242 418 A, US 6 261 264 A, US 5 472 430 A and CN 103 357 090 A disclose a syringe needle assembly with various designs, among which are elastic sheaths with deformable sections and/or telescopic arrangements. Document US 5 478 328A discloses a syringe needle assembly according to the preamble of claim 1.

### Detailed Description of the Invention

### Technical Problem

An object of the present invention is to provide a syringe needle assembly which is configured to be applicable to an existing syringe when used by upgrading the existing syringe without substantially changing the structure of the existing syringe, has a simple and safe structure, and is configured to be used easily and conveniently, and a safety syringe and a safety syringe device using the syringe needle assembly.

Another object is to provide an integral safety syringe through assembly with an available existing safety syringe for filtering fine glass powder of an ample, using compatibility which is an advantage of upgrading.

### Technical Solution

The objects described above are achieved by a syringe assembly according to claim 1.
The syringe needle assembly includes a syringe needle having a needle tip formed on the front end thereof, and a safety tube covering the syringe needle, coupled to a rear end of the syringe needle and opened in an injection direction of the syringe needle. When the syringe needle is inserted into skin, the safety tube is contracted towards a rear side of the needle tip, and when the syringe needle is pulled out from the skin, the safety tube is extended towards the front side of the needle tip.

The safety tube may be formed of rubber, a resin or a silicon material.

The safety tube includes a deformation part configured to be elastically deformed when the syringe needle is inserted into the skin or pulled out from the skin, and a fixing part configured to support the deformation part in the longitudinal direction of the syringe needle.

The deformation part may be formed of a soft flexible material, and the fixing part may be formed of a material harder than the deformation part.

The deformation part may be formed in a thickness Lhinner than the fixing part.

A deformation guide recess may be formed along a circumferential direction on an inner circumferential surface or an outer circumferential surface of the deformation part so that elastic deformation is intensively generated at the deformation guide recess.

The deformation part may be formed on the front end portion of the safety tube, and the front end portion of the deformation part may flare outward so that the inner circumferential surface is in contact with the skin.

The deformation part is formed to have an inner diameter which may increase or be constant in the injection direction of the syringe needle.

The deformation part may be formed on the middle portion of the safety tube and may have an inner diameter larger than the fixing part.

The deformation part may be formed on the middle portion of the safety tube, and a cutout may be formed on an inner circumferential surface or an outer circumferential surface of the deformation part.

The syringe needle assembly of the present invention further includes a slider formed to be movable along the outer circumferential surface of the safety tube. In a case where the slider part is positioned on the outer circumferential surface of the deformation part, the inner circumferential surface of the slider compresses the outer circumferential surface of the deformation part when the deformation part is elastically deformed.

At least one rib may be formed on the outer circumferential surface of the safety tube to form a boundary for a moving distance of the slider between the deformation part and the fixing part.

A rib may be formed on the inner circumferential surface, and an insertion recess or an insertion hole may be formed on the outer circumferential surface of the safety tube so that the rib is inserted into the insertion recess or the insertion hole to form a boundary for a moving distance of the slider between the deformation part and the fixing part.

An inclined member is formed on the front end portion of the safety tube. The inclined member has an outer diameter that increases along the injection direction of the syringe needle and an inner diameter that is reduced when the slider is positioned on an outer circumference of the inclined member.

An engagement recess or an engagement hole is formed on the slider, and a protrusion is formed on the front end portion of the safety tube to be inserted and engaged in the engagement recess or the engagement hole when the slider is positioned on the outer circumferential surface of the inclined member.

The safety tube may include a first tube, and a second tube slid along a longitudinal direction of the first tube.

The first tube and the second tube may be respectively formed with screw threads which are engaged with each other by relative rotation when the safety tube is extended.

One of the first tube and the second tube may be formed with a protrusion and the other may be formed with an insertion slot. When the safety tube is extended, the protrusion is inserted and engaged in the insertion slot so that the extended state of the safety tube is maintained.

One of the first tube and the second tube may be formed with a sliding rib, and the other may be formed with a sliding recess so that the sliding rib is inserted into the sliding recess to move along the sliding recess.

The sliding recess may include a moving section configured to allow a sliding movement of the second tube along the longitudinal direction of the first tube, and a limit section configured to limit the sliding movement of the second tube along the longitudinal direction of the first tube.

The objects described above may also be achieved by a safety syringe according to the present invention. The safety syringe includes a syringe needle assembly, a syringe barrel, to which the syringe needle assembly is coupled, and a piston configured to move within the syringe barrel.

The objects described above may also be achieved by a safety syringe device according to the present invention. The safety syringe device includes a syringe needle assembly, a syringe barrel, to which the syringe needle assembly is coupled, a piston configured to move within the syringe barrel, and a safety stand having an insertion hole formed in a longitudinal direction. The insertion hole has a size larger than the inner diameter of the slider and smaller than the outer diameter of the slider. When a lower end of the safety tube is inserted into the insertion hole in a state where the needle tip is directed upward, the slider is moved to the deformation part by the weight of the safety syringe.

### Advantageous Effects

According to the present invention, a syringe needle assembly, and a safety syringe and a safety syringe device which use the syringe needle assembly may be provided, in which the syringe needle assembly includes a syringe needle having a needle tip formed on the front end thereof and a safety tube covering the syringe needle, coupled to the rear end of the syringe needle and opened in the injection direction of the syringe needle. When the syringe needle is inserted into the skin, the safety tube is contracted towards the rear side of the needle tip, and when the syringe needle is pulled out from the skin, the safety tube is extended towards the front side of the needle tip. Thus, the syringe needle assembly, the safety syringe, and the safety syringe device of the present invention are simple in construction and may be safely and conveniently used while being applied to and used with an existing syringe by upgrading the existing syringe without changing the construction of the existing syringe.

### Brief Description of the Drawings

FIG. 1 is a view illustrating a safety syringe according to an embodiment of the present invention;
FIGS. 2a to 2c are views illustrating an applied state of the syringe needle assembly according to the embodiment of the present invention;
FIGS. 3a to 3d are views illustrating a syringe needle assembly according to another embodiment of the present invention;
FIGS. 4a to 4c are views illustrating a syringe needle assembly according to still another embodiment of the present invention;
FIGS. 5a to 5d are views illustrating a syringe needle assembly according to still another embodiment of the present invention;
FIG. 6 is a view illustrating a syringe needle assembly according to still another embodiment of the present invention;
FIGS. 7a to 7c are views illustrating a syringe needle assembly according to still another embodiment of the present invention;
FIGS. 8a and 8b are views illustrating a syringe needle assembly according to still another embodiment of the present invention;
FIG. 9 is a view illustrating a syringe needle assembly according to yet another embodiment of the present invention;
FIG. 10 is a view illustrating a syringe needle assembly according to yet another embodiment of the present invention;
FIG. 11 is a view illustrating a syringe needle assembly according to yet another embodiment of the present invention;
FIG. 12 is a view illustrating a syringe needle device according to yet another embodiment of the present invention; and
FIG. 13 is a view illustrating an applied state of the safety syringe device of FIG. 12.

### *Reference Numerals of Main Components in Drawings*

1: safety syringe device
10: safety syringe
30: safety stand
100: syringe needle assembly
200: syringe barrel
300: piston
110: syringe needle
150: slider
111: needle tip
151: engagement hole
113: coupling part
31: base
130: safety tube
33: support leg
131: deformation part
35: stand plate
133: fixing part
H: insertion hole
E: rib
Ta: narrowed part
S: inclined member
Tb: widened part
P: protrusion
R: sliding recess
N: cutout
R1: moving section
T1: first tube
R2: limit section
T2: second tube
V: sliding rib
T3: third tube
G: deformation guide recess
P2: protrusion
Th: screw thread
H1: insertion slot

### Mode for Carrying Out the Invention

Hereinafter, embodiments of the present invention will be described in detail with reference to the accompanying drawings. In the description of the present invention, the description of the well-known function or structure will be omitted in order to clear the subject matter of the present invention.

A syringe needle assembly, and a safety syringe and a safety syringe device using the syringe needle assembly are configured to be applicable to an existing syringe when used by upgrading the existing syringe without changing the existing syringe, have a simple and safe structure, and are configured to be used easily and conveniently.

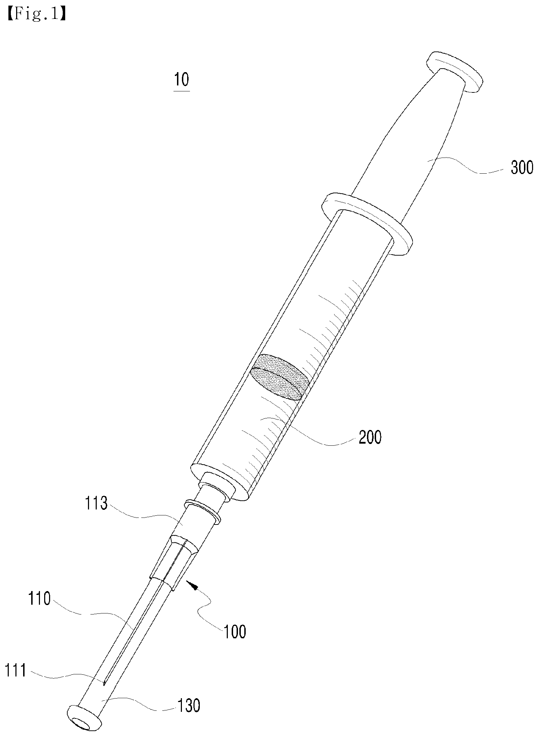

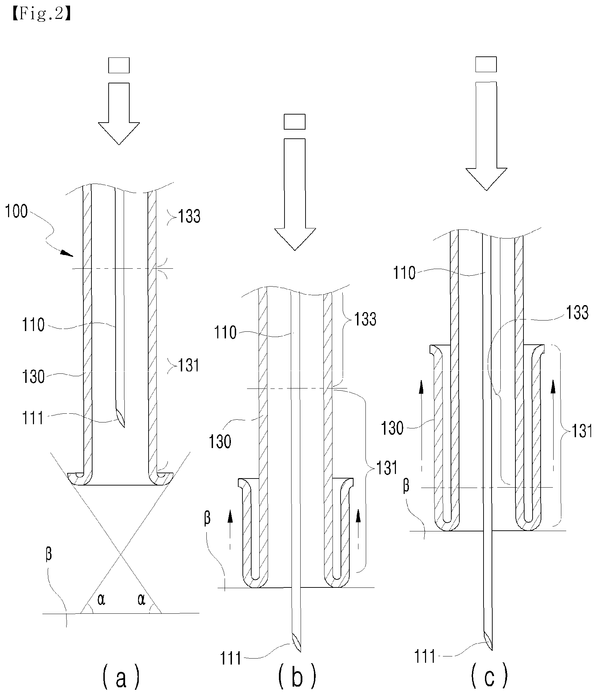

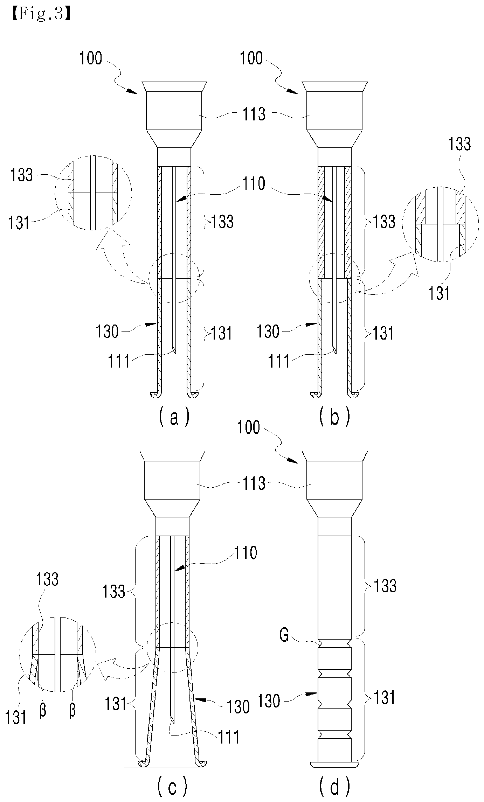

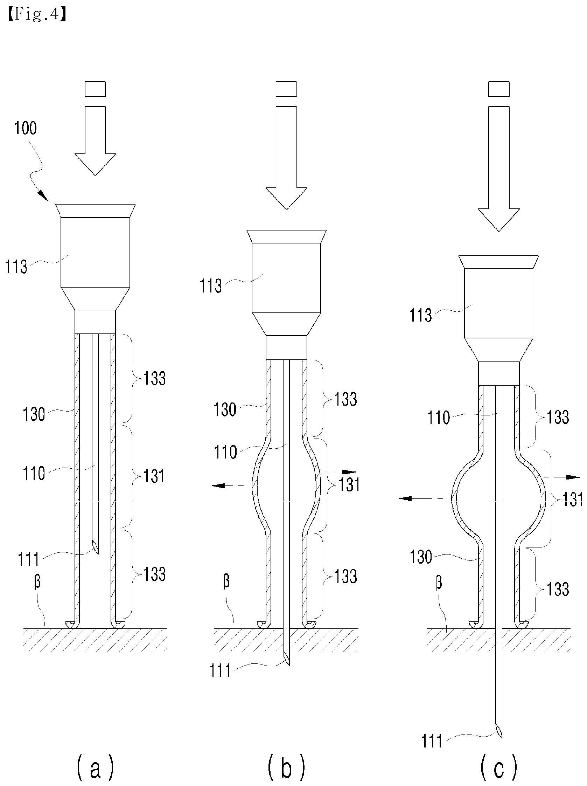

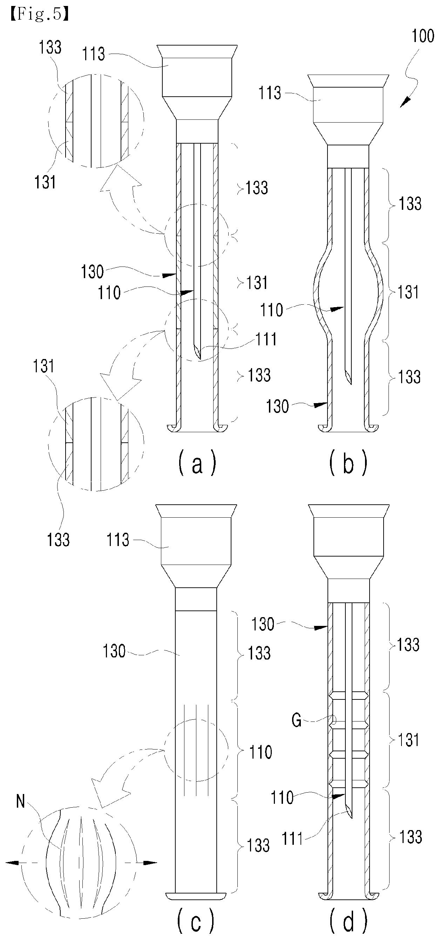

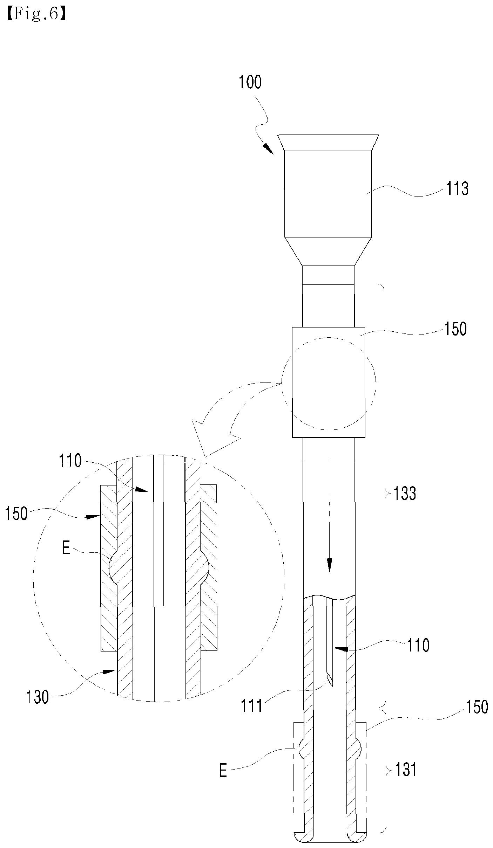

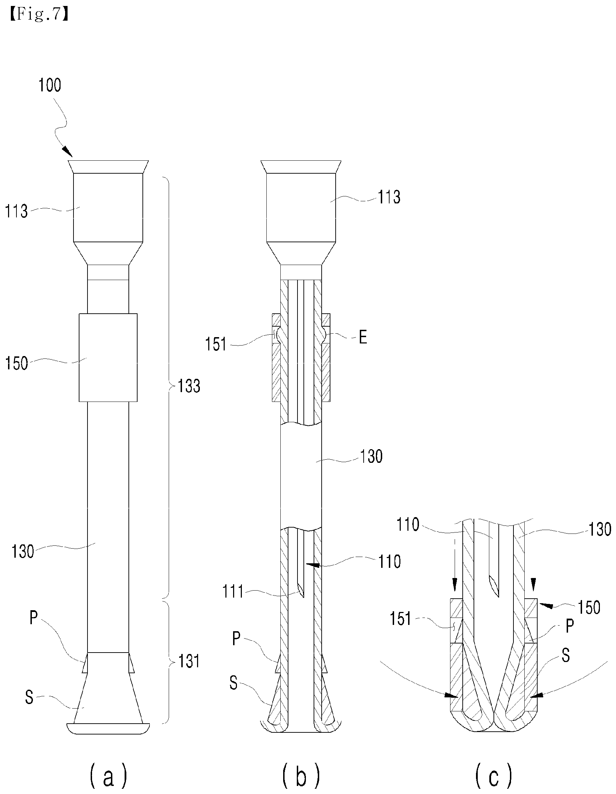

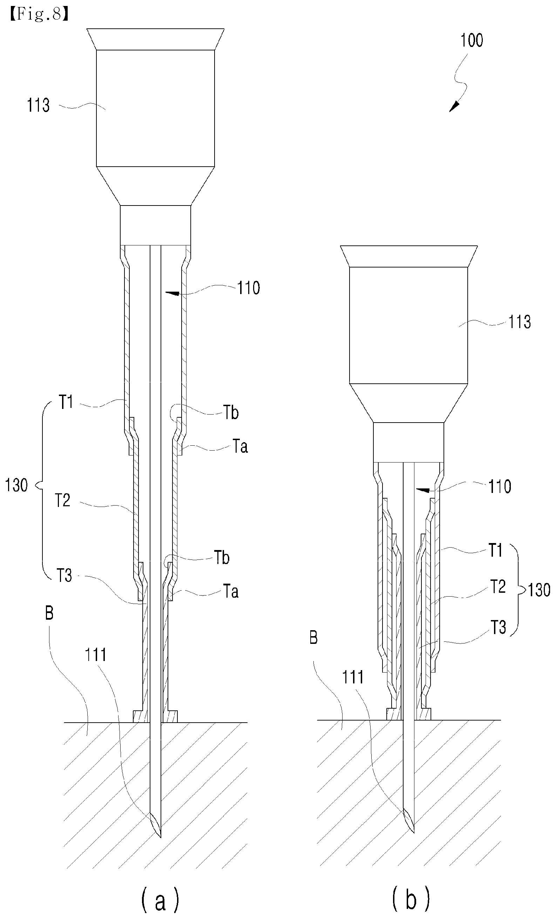

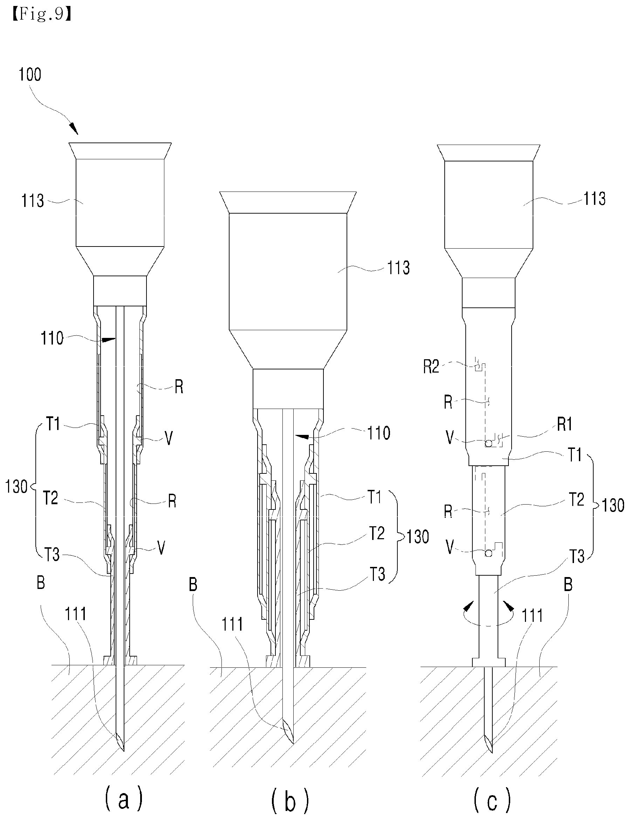

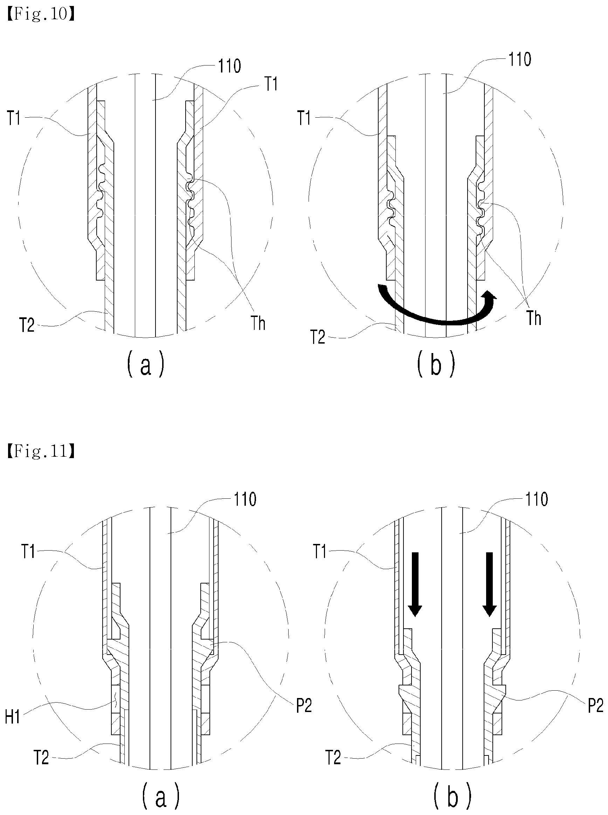

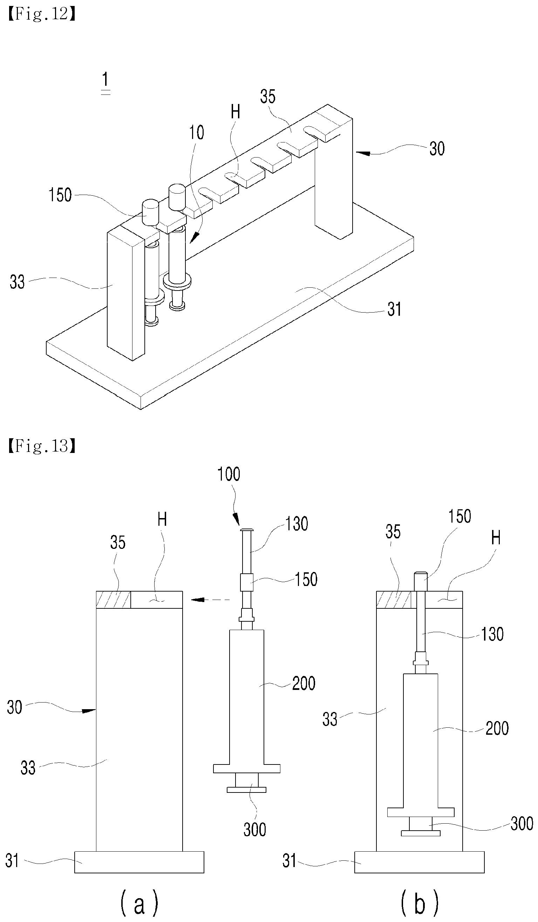

FIG. 1 is a view illustrating a safety syringe according to a first embodiment of the present invention, FIGS. 2a to 2c are views illustrating an applied state of the syringe needle assembly according to the first embodiment of the present invention, and FIGS. 3a to 3d are views illustrating a syringe needle assembly according to a second embodiment of the present invention. FIGS. 4a to 4c are views illustrating a syringe needle assembly according to a third embodiment of the present invention, FIGS. 5a to 5d are views illustrating a syringe needle assembly according to a fourth embodiment of the present invention, and FIG. 6 is a view illustrating a syringe needle assembly according to a fifth embodiment of the present invention. FIGS. 7a to 7c are views illustrating a syringe needle assembly according to a sixth embodiment of the present invention, FIGS. 8a and 8b are views illustrating a syringe needle assembly according to a seventh embodiment of the present invention, and FIG. 9 is a view illustrating a syringe needle assembly according to a seventh embodiment of the present invention. FIG. 10 is a view illustrating a syringe needle assembly according to an eighth embodiment of the present invention and FIG. 11 is a view illustrating a syringe needle assembly according to a ninth embodiment of the present invention. FIG. 12 is a view illustrating a syringe needle device according to a tenth embodiment of the present invention, and FIG. 13 is a view illustrating an applied state of the safety syringe device of FIG. 12.

As illustrated in FIG. 1, a safety syringe 10 according to a first embodiment is configured to prevent cross infection by a syringe needle 100 contaminated with a patient's blood after injection, and includes a syringe needle assembly 100, a syringe barrel 200, and a piston 300. The syringe barrel 200 and the piston 300 are the same as those of well-known syringes, and thus, detailed descriptions thereof will be omitted.

The syringe needle assembly 100 includes a syringe needle 110 and a safety tube 130, and is integrally or removably coupled to the syringe barrel 200. In the first embodiment of the present invention, the syringe needle assembly 100 will be described assuming that it is removably coupled to the syringe barrel 200.

As illustrated in FIG. 3, the syringe needle 110 has a needle tip 111 formed on the front end thereof like an ordinary syringe needle, and a coupling part 113 formed on the rear end thereof to be coupled to the syringe barrel 200. Although not illustrated, as for the coupling structure between the coupling part 113 and the syringe barrel 200, a well-known structure between the syringe barrel and the syringe needle, such as a luer lock coupling structure, may be variously applied.

The safety tube 130 is configured to cover the syringe needle 110, in which the safety tube 130 is coupled to the rear end of the syringe needle 110 and opened in the injection direction of the syringe needle 110. More specifically, the safety tube 130 is formed in a cylindrical tube having an inner diameter which is larger than the outer diameter of the syringe needle 110, and the rear end of the safety tube 130 is coupled to the syringe needle 110 in a state where the syringe needle 110 is inserted therein.

In the state where the safety tube 130 is covering the syringe needle 110, the inner circumferential surface of the safety tube 130 is spaced apart from the syringe needle 110, except for the rear end thereof. The front end of the safety tube 130 extends beyond the front end of the syringe needle 110 by a predetermined distance.

The safety tube 130 is made of an elastically deformable rubber, resin, or silicon, so that when the syringe needle 110 penetrates skin B, the safety tube 130 is contracted backward form the needle tip 111 due to its own elasticity, and when the syringe needle 110 is pulled out from the skin B, the safety tube 130 is expanded to the front side of the needle tip 111 by the elastic restoration thereof. Preferably, the safety tube 130 is made of a biomedical polymer material.

Preferably, the safety tube 130 is formed to be transparent so that medical personnel are capable of observing the syringe needle 110 therein.

Of course, the safety tube 130 may be formed to be opaque or colored so that the syringe needle 110 is not observed. When the syringe needle 110 is not observed due to the safety tube 130, there is an advantage in that fear experienced by children or patients with needle phobia can be mitigated.

As illustrated in FIGS. 2 and 4, the front end of the safety tube 130 comes into contact with the surface of the skin first before the syringe needle 110 comes into contact with the skin B at the time of injection, and as the syringe needle 110 is moved towards the skin B, a larger contractile force acts on the safety tube 130.

The contractile force acting on the safety tube 130 gradually increases as the linear distance between the rear end of the safety tube 130 and the skin B while the syringe needle 110 penetrates the surface of the skin B, and thus the safety tube 130 is elastically contracted along the longitudinal direction.

Although not illustrated, when the syringe needle 110 completely penetrates the skin B, the piston 300 is pushed so that the liquid substance contained in the syringe barrel 200 is introduced into a blood vessel or the like. Of course, when collecting blood, the liquid inside of the skin B, such as blood, may be drawn into the syringe barrel 200 when the piston 300 is pulled.

When introduction of a liquid substance or collection of blood is completed, the syringe needle 110 is pulled out from the skin B in the direction opposite to the injection direction. While the syringe needle 110 is pulled out, the contractile force acting on the safety tube 130 gradually decreases as the syringe needle 110 moves. Thus, the safety tube 130 elastically expands along the longitudinal direction.

The gradual expansion of the safety tube 130 continues until the contact between the front end of the safety tube 130 extending beyond the syringe needle 110 and the skin B is released after the syringe needle 110 is completely pulled out from the skin B.

When the introduction of liquid substance or the blood collection is completed, the syringe needle is stained with a very small amount of the patient's blood. In the past, the syringe needle was covered with a stopper and discarded in order to prevent medical personnel, such as a physician, nurse, or a janitor from being pricked by the syringe needle.

The syringe needle assembly 100 of the present invention solves the problems as described above by covering the safety tube 130 over the syringe needle 110. That is, the syringe needle 110 is always maintained in the state where it is covered by the safety tube 130. Accordingly, even when the syringe needle 110 side portion is gripped with a hand or the syringe needle 110 is inadvertently brought into contact with the skin B, the hand or the skin B is always brought into contact with the safety tube 130 first. Thus, it is possible to prevent the skin or the hand from being pricked by the syringe needle 110.

Referring to FIGS. 2 to 5, the safety tube 130 may include a deformation part 131 that is elastically deformed when the syringe needle 110 penetrates the skin B or is pulled out from the skin B, and a fixing part 133 that supports the deformation part 131 in the longitudinal direction of the syringe needle 110. The deformation part 131 may be formed on the front portion, the middle portion or the rear portion of the safety tube 130 in the longitudinal direction.

As illustrated in FIG. 2a, the deformation part 131 may be formed on the front end portion of the safety tube 130 and the front end of the deformation part 131 may flare outward to be in contact with the skin B.

At this time, when a flaring-outward angle α of the front end portion of the deformation part 131 is formed, the deformation part 131 to be in contact with the skin B at the time of injection is widened from the front end portion (see FIG. 2b) and as the syringe needle 110 is moved in the injection direction, the portion contacted with the skin B is continuously curled up so that the length of the deformation part 131 is reduced (see FIG. 2c).

When the syringe needle 110 is pulled out from the skin B, the curled portion deformation part 131 formed of an elastic flexible material is unrolled to be returned to the original position by the elastic restoration thereof.

The injection direction, in which the syringe needle 110 penetrates the skin B, may perpendicular to the skin surface but may be inclined by a predetermined angle with respect to the skin surface according to an injection purpose.

Although not illustrated, when the front end portion of the deformation part 131 forms the flaring-outward angle α, the inner circumferential surface of the deformation part 131 comes in contact with the skin B first even if the syringe needle 110 is injected in the inclined direction with respect to the skin surface. Accordingly, there is an advantage in that the deformation part 131 is easily deformed outwardly from the front end thereof by the force applied to the inner circumferential surface thereof by the skin B in a direction substantially opposite to the injection direction.

As illustrated in FIG. 3, each of the deformation part 131 and the fixing part 133 forms a predetermined section along the longitudinal direction of the safety tube 130 and the deformation part 131 and the fixing part 133 may be differentiated from each other in the following ways.

As illustrated in FIG. 3a, the deformation part 131 may be made of a soft flexible material, and the fixing part 133 may be made of a material harder than the deformation part 131. That is, since the deformation part 131 is made of a more flexible material than the fixing part 133, the deformation part 131 is deformed first by a contractile force acting in the longitudinal direction of the safety tube 130 at the time of injection for introduction of a liquid substance, blood collection or the like.

Since the fixing part 133 is made of a harder material than the deformation part 131, the fixing part 133 supports the deformation part 131 in the longitudinal direction of the syringe needle 110 when the deformation part 131 is contracted. The fixing part 133 may be made of a non-flexible material but may be made of a flexible material harder than the deformation part 131.

As illustrated in FIG. 3b, the deformation part 131 may be formed in a thickness thinner than the fixing part 133. When the deformation part 131 is formed in a thickness thinner than the fixing part 133, the expansion or contraction of the deformation part 131 advances first when the contractile force acts on the safety tube 130, and when the deformation part 131 is contracted, the fixing part 133 supports the deformation part 131 in the longitudinal direction.

As illustrated in FIG. 3c, the deformation part 131 may be formed such that its inner diameter increases or is constant in the injection direction of the syringe needle 110. When the inner diameter of the deformation part 131 made of the flexible material increases along the injection direction by a predetermined angle β, there is an advantage in that the front end of the deformation part 131 may be smoothly curled in proportion to the angle β even with a smaller contractile force since the inner diameter of the deformation unit 131 decreases towards the rear end of the deformation unit 131 by the predetermined angle β while the deformation part 131 is curled outward from the front end thereof.

In addition, when the deformation part 131 has an inner diameter increasing along the injection direction by the predetermined angle β, the deformation part 131 may be smoothly curled even with a smaller contractile force even if the syringe needle 110 is injected in the inclined direction with respect to the skin B, so that when performing the injection, the safety tube 130 can be prevented in advance from covering the syringe needle 110 in the curled state.

As illustrated in FIG. 3d, a deformation guide recess G may be formed on the outer circumferential surfaces of the deformation part 131 to extend in the circumferential direction so that an elastic deformation may be intensively generated at the deformation guide recess G. That is, when the deformation guide recess G is formed to reduce the thickness of the deformation part 131, the deformation part 131 of the safety tube 130 can be subjected to a relative large elastic deformation at the deformation guide recesses G.

In addition, it is preferable that one or more deformation guide recesses G are formed from the front end of the deformation part 131. It is also preferable that the deformation guide recesses G are repeatedly formed at predetermined intervals along the longitudinal direction of the safety tube 130.

As illustrated in FIGS. 4a to 4c, the deformation part 131 may be formed in the middle portion of the safety tube 130 to have an inner diameter larger than the fixing part 133.

When the deformation part 131 is formed in the middle portion of the safety tube 130, fixing parts 133 are formed on opposite end sides of the deformation part 131, i.e., in the front and rear end portions of the safety tube 130, so that when giving an injection, the front end of the fixing part 133 formed in the front end portion of the safety tube comes into contact with the skin B where the syringe needle 110 penetrates. When the contractile force acts on the safety tube 130, the deformation part 131 is contracted while being deformed in an outwardly expanded shape over the longitudinal direction thereof.

As illustrated in FIG. 5a, the deformation part 131 may be made of a soft flexible material and the fixing parts 133 may be made of a material harder than the deformation part 131. That is, as the deformation part 131 is formed of a flexible material softer than the fixing part 133, the deformation part 131 is deformed first by the contractile force acting in the longitudinal direction of the safety tube 130 while giving an injection for introducing a liquid substance, collecting blood or the like.

The fixing parts are formed of a material harder than the deformation part 131 to support the deformation part 131 on the opposite sides of the deformation part 131 when the deformation part 131 is expanded or contracted. Although the fixing part 133 may be formed of a non-flexible material, the deformation part 131 may be formed of a harder flexible material.

As illustrated in FIG. 5b, the deformation part 131 may be formed to have an inner diameter increasing towards its longitudinal central portion or a constant diameter. When the deformation part 131 formed of a flexible material has an inner diameter increasing towards its longitudinal central portion, the deformation part 131 may be smoothly deformed even with a smaller contractile force.

As illustrated in FIG. 5c, cutouts N may be formed on the inner or outer circumferential surface of the deformation part 131. When the cutouts N are formed on the deformation part 131, the internal restraint force of the deformation part is reduced in a direction inclined to the injection direction, so that the deformation part 131 may be smoothly deformed even with a smaller contractile force.

As illustrated in FIG. 5d, a deformation guide recess G may be formed on the inner circumferential surface of the deformation part 131 along the circumferential direction so that the elastic deformation is intensively generated at the deformation guide recess G. That is, when the deformation guide recess G is formed on the deformation part 131 of the safety tube 130 to the thickness of the deformation part 131, a relatively large elastic deformation may be generated at the deformation guide recess G.

In addition, one deformation guide recess G may be formed on the longitudinal central portion of the deformation part 131, or two or more deformation guide recesses G may be repeatedly formed at predetermined intervals along the longitudinal direction of the safety tube 130 with reference to the longitudinal center of the deformation part 131.

Although not illustrate, the deformation part 131 may of course be formed to have a thickness thinner than the fixing part 133.

As illustrated in FIG. 6, according to another embodiment of the present invention, the syringe needle assembly 100 may further include a slider 150. Although FIG. 6 illustrates a syringe needle assembly 100 in which the deformation part 131 is formed in the front end portion of the safety tube 130, the slider 150 may of course also be applied to a syringe needle assembly 100 in which the deformation part 131 is formed in the middle portion or the rear end portion of the safety tube 130.

The slider 150 is a component to restrain the outer circumferential surface of the deformation part by the inner circumferential surface to prevent the elastic deformation of the deformation part 131 when giving an injection. The slide 150 is formed to be movable along the outer circumferential surface of the safety tube 130.

When the slider 150 is positioned on the outer circumferential surface of the deformation part 131, the expansion/retraction of the deformation part 131 is prevented even if a contractile force acts on the safety tube 130. Accordingly, the needle tip 111 of the syringe needle 110 cannot protrude out in the injection direction from the inside of the safety tube 130.

Meanwhile, in the case where the slider 150 is positioned on the outer circumferential surface of the fixing part 133, when the slider 150 is located at a position where the slider 150 does not affect the expansion/contraction of the deformation part 131, the deformation part 131 may be expanded/contracted when performing the injection so that the syringe needle 110 may penetrate into the skin B.

Accordingly, before or after the safety syringe 10 is used, the slider 150 may be positioned on the outer circumferential surface of the deformation part 131, and when performing the injection, the slider 150 may be slid onto the outer circumferential surface of the fixing part 133 and then the injection may be performed. Thus, when the injection is not performed, an accident caused by being pricked by the syringe needle 110 may be fundamentally prevented.

One or more ribs E may be provided on the outer circumference of the safety tube 130 to form a boundary for the moving distance of the slider 150 between the deformation part 131 and the fixing part 133. When both the fixing part 133 and the deformation part 131 are formed with the rib E, the reciprocating distance of the slider 150 between the fixing part 133 and the deformation part 131 may be set, and when the slider 150 is fitted on the rib E, the position of the slider 150 may be fixed. On the internal circumferential surface of the slider 150, a coupling recess or engagement hole 151 may be formed so that the rib E may be fitted therein.

As illustrated in FIGS. 7a and 7c, an inclined member S may be formed in the front end portion of the safety tube 130, in which the inclined member S has an outer diameter increasing along the injection direction of the syringe needle 110 and the inner diameter of the inclined member S decreases when the slider 150 is positioned on the outer circumferential surface of the inclined member S.

The inclined member S is fitted on the outer circumferential of the safety tube 130. Of course, the inclined member S may be integrally formed with the safety tube 130.

As illustrated in FIG. 7c, in the case where the inclined member S, of which the outer diameter increases along the injection direction, is formed on the front end portion of the safety tube 130, when the slider 150 is slid onto the outer circumferential surface of the inclined member S, the inclined member S and the front end portion of the safety tube 130 are pressed to the inside of the safety tube 130 by the inner circumferential surface of the slider 150.

Upon being elastically contracted circumferentially and to the inside of the safety tube 130 by the compressive force by the inner circumferential surface of the slider 150, the inclined member S and the front end portion of the safety tube 130 block the inside of the front end portion of the safety tube 130 where the syringe needle 110 protrudes.

When the inside of the front end portion of the safety tube 130 is blocked, accidents caused when medical personnel are pricked by the used syringe needle 110 can be perfectly prevented.

It is preferable that the inclined member S and the front end portion of the safety tube 130 are formed of a flexible material to be contacted in the circumferential direction and to the inside of the safety tube 130 by the compressive force of the slider 150. Of course, the inclined member S may be formed of a hard material.

As illustrated in FIG. 7a, the inclined member S may be formed to have a predetermined inclination in the circumferential direction. Alternatively, the inclined member S may be formed by a pair of sections positioned on opposite sides and having symmetric inclinations, three sections with an included angle of 60 degrees, or four sections with an included angle of 90 degrees (not illustrated).

In addition, when the inclined member S is formed on the front end portion of the safety tube 130, and the deformation part 131 is formed between two fixing parts 133, the force applied to the front end portion of the safety tube 130 while coming into contact with the skin B is transferred to the deformation part 131 in the state where the shape of the front end portion is maintained by the inclined member S even if the syringe needle 110 is injected in the inclined direction with respected to the skin B. Thus, the safety tube 130 may be reliably deformed while forming a rigid force transfer structure.

As illustrated in FIGS. 7b and 7c, in the case where the coupling recess or engagement hole 151 is formed on the slider 150, and the slider 150 is positioned on the outer circumferential surface of the inclined member S on the front end portion of the safety tube 130, a protrusion P may be formed to be fitted in the coupling recess or engagement hole 151.

The protrusion P is formed to protrude more outwardly along the injection direction. When the protrusion P is fitted in the engagement hole 151 of the slider 150, the movement of the slider 150 towards the rear end of the safety tube 130 is blocked so that the inside of the safety tube 130 is continuously maintained in the blocked state. Thus, the probability of reusing the syringe needle 110 can be blocked.

As illustrated in FIG. 8, according to still another embodiment of the present invention, a syringe needle assembly 100 may include a safety tube 130 including a first tube T1, a second tube T2 sliding along the longitudinal direction of the first tube T1, and a third tube T3 sliding along the longitudinal direction of the second tube T2. The safety tube 130 is not necessarily limited to the three-stage configuration of the first tube T1, the second tube T2, and the third tube T3 and may be formed in a two-stage configuration of the first tube T1 and the second tube T2. Of course, although not illustrated, the safety tube 130 may be formed in a four or more-stage configuration.

The safety tube 130 is normally maintained in the state where it is covering the syringe needle 110 (see FIG. 8a), and when giving an injection, the front end of the third tube T3 is in contact with the skin B and slid along the longitudinal direction of the second tube T2, and as the syringe needle 110 penetrates the skin B, the first tube T1, the second tube T2, and the third tube T3 are sequentially retracted (see FIG. 8b).

In order to prevent each of the safety tubes T1, T2 and T3 from being released from an adjacent safety tube T1, T2 or T3, engagement structures Ta and Tb are formed on two adjacent safety tubes T1 and T2; T2 and T3, respectively. That is, a narrowed part Ta is formed in the front end portion of each of the first tube T1 and the second tube T2, and a widened part Tb is formed in the rear end portion of each of the second tube T2 and the third tube T3.

As a result, when the safety tube 130 is extended, the outer circumferential surface of the widened part is engaged with the inner circumferential surface of the narrowed part so that the second tube T2 and the third tube T3 are prevented from being released from the first tube T1 and the second tube T2, respectively.

At the terminal end of the third tube T3 positioned at the front end portion forming a free end among the plurality of safety tubes T1, T2 and T3, an engagement part is provided which is formed to be enlarged radially outwardly. By the engagement part, the third tube T3 positioned at the front end portion is prevented from being completely introduced into the second tube T2.

Although not illustrated, the third tube T3 disposed at the front end among the safety tubes 130 to come in contact with the skin may be formed of a plastically deformed material. In a case where the third tube T3 is formed of a plastically deformed material, when the middle portion of the third tube T3 is bent after the syringe needle 110 is used, the protrusion of the syringe needle 110 through the third tube T3 is blocked and the inside of the safety tube 130 is continuously maintained in the blocked state. Thus, the probability of reusing the syringe needle 110 can be blocked.

As illustrated in FIG. 9, according to still another exemplary embodiment of the present invention, the syringe needle assembly 100 may include a sliding rib V formed on one of the first tube T1 and the second tube T2 and a sliding recess R formed on the other of the first tube T1 and the second tube T2 so that the sliding rib V is inserted into and moved along the sliding recess R. Of course, when the safety tube 130 is formed in three stages, a sliding rib V is also formed in one of the second tube T2 and the third tube T3 and a sliding recess R is also formed on the other of the second tube T2 and the third tube T3. In the following, descriptions will be made assuming that the sliding rib V and the sliding recess R are formed on the first tube T1 and the second tube T2. Even if the safety tube 130 is formed in three or more stages, the sliding rib V and the sliding recess R are similarly configured.

When the sliding rib V is formed on the inner circumferential surface of the first tube T1, the sliding recess R is formed on the outer circumferential surface of the second tube T2, and when the sliding recess R is formed on the inner circumferential surface of the first tube T1, the sliding rib V is formed on the outer circumferential surface of the second tube T2. The latter case is illustrated in FIG. 9.

As described above, the number of sliding ribs V and sliding recesses R are varied depending on the multi-stage configuration of the safety tubes T1, T2 and T3 which are configured not to be piled up one on another in the routes thereof. As illustrated in FIG. 9, when three safety tubes T1, T2 and T3 are provided, two sliding ribs V and two sliding recesses R are formed. The sliding ribs V and the sliding recesses R may be bisected T2 and T3 to be provided at 180 degree intervals or quartered to be provided at 90 degree intervals along the circumferential direction of the safety tubes T1.

Each sliding recess R includes a moving section R1 and a limit section R2.

The moving section R1 refers to a section that allows sliding of the second tube T2 along the longitudinal direction of the first tube T1, and the limit section R2 refers to a section that limits the sliding of the second tube T2 along the first tube T1.

Specifically, the moving section R1 is formed parallel to the longitudinal direction of the first tube T1 so as to allow the second tube T2, which is integrally formed with the sliding rib V, to be slid along the longitudinal direction of the first tube T1. The limit section R2 is formed to be curved from the moving section R1 so as to limit the movement of the second tube T2, which is integrally formed with the sliding rib V, along the longitudinal direction of the first tube T1.

The limit section R2 may be formed in a shape which is merely curved (preferably in an orthogonal direction) from the end of the moving section R1. As illustrated in FIG. 9, the limit section R2 may be dually curved so that the sliding rib V is locked at the end of the limit section R2.

As illustrated in FIG. 9, the limit section R2 may be formed on each of the upper and lower ends of the moving sections R1 or at any one of the upper and lower ends. When the limit section R2 is formed on each of the upper and lower ends of the moving section R1, the limit sections R2 may be formed to be directed in the same direction or in the opposite directions as illustrated in FIG. 9.

In addition, the limit section R2 may be formed on the left or right side in the corresponding end of the moving section R1, and as a result, the second tube T2 may be rotated clockwise or counterclockwise in relation to the first tube T1.

Although not illustrated, the sliding ribs V may serve as a handle to be gripped when extending or contracting the safety tube 130.

That is, the sliding recesses R may be formed through the first tube T1 and the second tube T2, and the sliding ribs V, which are respectively formed on the second tube T2 and the third tube T3, may be formed to protrude beyond the outer circumferential surfaces of the first tube T1 and the second tube T2 through the sliding recesses R so that the sliding ribs V may be gripped instead of the first tube T1 and the second tube T2 to extend or contract the safety tube 130.

As illustrated in FIGS. 10a and 10b, according to still another embodiment of the present invention, an syringe needle assembly 100 may include screw threads Th formed on the first tube T1 and the second tube T2, respectively, to be engaged with each other by relative rotation of the first tube T1 and the second tube T2 when extending the safety tube 130.

The second tube T2 is freely slid along the longitudinal direction of the first tube T1 until the syringe needle assembly 100 is used, and the first tube T1 and the second tube T2 are screw-engaged with each other after the syringe needle assembly 100 is used, that is, when the syringe needle assembly 100 is discarded.

The screw thread Th of the first tube T1 is formed on the inner circumferential surface of the front end portion of the first tube T1 in the injection direction and the screw thread Th of the second tube T2 are formed on the outer circumferential surface of the rear end of the second tube T2 in the injection direction. Accordingly, the screw threads Th of the first tube T1 and the second tube T2 are in contact with each other in a state where the safety tube 130 is extended towards the front side of the needle tip 111 of the syringe needle 110.

Since the first tube T1 is fixed to a coupling part 113 not to be rotated, when medical personnel rotate the second tube T2 in the direction where a pair of the screw threads Th are to be engaged with each other, the rectilinear relative movement between the first tube T1 and the second tube T2 is blocked by the engagement force between the screw threads Th in the state where the safety tube 130 is extended towards the front side of the needle tip 111 of the syringe needle 110.

Although not illustrated, when the safety tube 130 is configured in three stages, screw threads Th are further formed on the second tube T2 and the third tube T3, respectively, to be engaged with each other when the second tube T2 and the third tube T3 are relatively rotated to extend the safety tube 130. At this time, when screw threads Th are formed such that the screw-engagement direction between the first tube T1 and the second tube T2 and the screw-engagement direction between the second tube T2 and third tube T3 are equal to each other, the first tube T1, the second tube T2, and the second tube T3 may be simultaneously screw-engaged with each other through a simple operation of rotating the third tube T3 in the screw-engagement direction in the state where the rotating safety tube 130 is extended towards the front side of the needle tip 111 of the syringe needle 110. The above-mentioned screw engagement structure may be equally applied when the safety tube 130 is formed in four or more stages.

As illustrated in FIGS. 11a and 11b, a syringe needle assembly 100 according to a still another embodiment may include a protrusion P2 formed in one of the first tube T1 and the second tube T2, and an insertion slot H1 formed in the other of the first tube T1 and the second tube T2 so that when the safety tube is extended, the protrusion P2 is inserted into the insertion slot H1. The following description will be made assuming that the protrusion P2 is formed in the second tube T2 and the first tube T1 is formed in the insertion slot H1.

The second tube T2 is freely slid along the longitudinal direction of the first tube T1 until the syringe needle assembly 100 is used, and the protrusion P2 and the insertion slot H1 are coupled to each other after the syringe needle assembly 100 is used, that is, when the syringe needle assembly 100 is discarded.

The insertion slot H1 of the first tube T1 is formed on the front end of the first tube T1 in the insertion direction and the protrusion P2 of the second tube T2 is formed on the front end of the second tube T2 in the injection direction so that protrusion P2 and the insertion slot H1 approach to each other as the safety tube 130 is extended towards the front side of the needle tip 111 of the syringe needle 110 more and more.

The protrusion P2 is formed to have an outer diameter larger than the inner diameter of the portion of the first tube T1 where the insertion slot H1 is formed, and thus, the protrusion P2 is not coupled to the insertion slot H1 unless a predetermined magnitude is applied in the coupling direction. When medical personnel pull the second tube T2 in the direction of extending the safety tube 130, the protrusion P2 is moved towards and coupled to the insertion slot H1 due to the radial elastic deformation of the first tube T1 and the second tube T2. As a result, in the state where the safety tube 130 is extended towards the front side of the needle tip 111 of the syringe needle 110, the rectilinear relative movement between the first tube T1 and the second tube T2 is blocked.

Preferably, the protrusion P2 protrudes from the outer circumferential surface of the second tube T2 in a right triangle shape or the like which has an inclined surface at the needle tip 111 side and a right surface at the coupling part 113 side so that the protrusion P2 may be easily inserted into the insertion slot H1 in the direction where the safety tube is extended and after inserted into the insertion slot H1, the protrusion P2 is prevented from being released from the insertion slot H1.

Although not illustrated, when the safety tube 130 is configured in three stages, another protrusion P2 is formed in any one of the second tube T2 and another insertion slot H1 is formed in the third tube T3 so that the protrusion P2 is inserted into the insertion hole H1 when the safety tube is extended. When the safety tube 130 is configured in four or more stages, still another protrusion P2 and still another insertion slot H1 which are the same as those described above are also formed in two adjacent tubes, respectively.

As illustrated in FIGS. 12 and 13, a safety syringe device 1 according to still another embodiment of the present invention includes a safety syringe 10 to which a slider 150 is coupled, and a safety stand 30 on which the safety syringe 10 is mounted after having been used.

As described above, when injection of a liquid substance, collection of blood or the like is completed, the syringe needle is stained with a small amount of a patient's blood. In the past, the syringe was discarded after covering the syringe needle with a stopper in order to prevent medical personnel such as a physician, nurse or a janitor from being pricked by the syringe needle.

The syringe needle assembly 100 of the present invention solves the problems as described above by covering the syringe needle 110 with the safety tube 130, and after the safety syringe 10 is used, by moving the slider 150 onto the deformation part 131 of the safety tube 130. However, since the slider 150 moves on the safety tube 130 adjacent to the syringe needle 110, the user may be pricked by the syringe needle 110 although it is highly unlikely when unexpected strong impact is applied externally in the process of holding the slider 150 with a finger so that the user's skin B of, for example, a hand, is intensely pressed against the syringe needle 110.

The safety syringe device 1 of the still another embodiment of the present invention includes a safety stand 30 capable of moving the slider 150 of a used safety syringe 10 onto the deformation part 131 without using a hand simultaneously with mounting the used safety syringe 10 on the safety stand 30, thereby completely excluding the possibility that the user's skin B of, for example, the hand, may be pricked by the syringe needle 110.

The safety stand 30 includes a base 31, support legs 33, and a stand plate 35.

The base 31 is provided as a bottom component provided so as to safely place the safety stand 30 on a top plate of a desk or the like. On the bottom surface of the base 31, a pad or the like formed of rubber or silicon may be assembled to provide friction safety so that the stand 30 does not slide on the top plate or the desk or the like, or a double-sided tape may be attached so that the stand 30 may be fixed to the top plate of the desk or the like.

The support legs 33 are components provided so as to support the stand plate 35 to be spaced apart from the base 31 in which a plurality of safety syringes 10 are arranged along the longitudinal direction of the of the stand plate 35 and supported by the stand plate 35. Preferably, a pair of support legs 33 are provided to be assembled to the both ends of the stand plate 35, respectively.

The stand plate 35 is a component provided to support the safety syringes 10 while being in contact with syringe needle assemblies 100, and insertion holes H are formed in the stand plate 35 so that when the syringe needle assemblies 100 are inserted, the insertion holes H come in contact with the sliders 150 of the syringe needle assemblies 100. The stand plate 35 is spaced apart from the base 31 by the support legs 33 at a height which is not less than the length of the safety syringes 10.

The insertion holes H are formed to be spaced apart from each other along the longitudinal direction and has a size larger than the inner diameter of the sliders 150 and smaller than the outer diameter of the sliders 150. The insertion holes H are formed to extend inwardly from a longitudinal edge of the stand plate 35 such that the safety tubes 130 of the syringes 10 may be horizontally inserted therein in the state where the safety syringes 10 stand vertically with the syringe needles 110 being directed upward.

Referring to FIG. 13, the safety syringe device 1 may be used as follows.

For example, when injection of a liquid substance into a patient's body or blood collection from the patient's body using a safety syringe 10 is completed, the safety syringe 10 is moved to the safety stand 30.

The safety tube 130 is inserted into an insertion hole H in the horizontal direction in the state where the safety syringe 10 stands vertically with the syringe needle 110 being directed upwardly. The portion between the slider 150 and the coupling part 113 of the syringe needle 110 on the safety tube 130 is inserted into the insertion hole H such that the slider 150 is positioned above the insertion hole H.

When medical personnel place the safety syringe 10 in the state where the safety tube 130 is inserted into the insertion hole H, the slider 150 is moved to the deformation part 131 by the weight of the safety syringe 10 in the state where the lower end of the slider 150 is in contact with the peripheral edge of the insertion hole H.

When the rib E or the protrusion P is formed on the deformation part 131, a downward external force is applied to the body portion of the safety syringe 10 so as to cause the rib E or the protrusion P to be engaged in the engagement hole 151 of the slider 150.

As described above, when the safety syringe device 1 is used, the probability of the user's skin B of, for example, a hand, being pricked by the syringe needle 110 may be completely removed.

According to the present invention, a syringe needle assembly 100, and a safety syringe 10 and a safety syringe device 1 which use the syringe needle assembly 100 may be provided, in which the syringe needle assembly 100 includes a syringe needle 110 having a needle tip 111 formed on the front end thereof and a safety tube 130 covering the syringe needle 110, coupled to the rear end of the syringe needle 110 and opened in the injection direction of the syringe needle 110. When the syringe needle 110 is inserted into the skin B, the safety tube 130 is contracted towards the rear side of the needle tip 111, and when the syringe needle 110 is pulled out from the skin B, the safety tube 130 is extended towards the front side of the needle tip 111. Thus, the syringe needle assembly 100, the safety syringe 10, and the safety syringe device 1 of the present invention are simple in construction and may be safely and conveniently used while being applied to and used with an existing syringe by upgrading the existing syringe without changing the construction of the existing syringe.

Although the exemplary embodiment of the present invention is described and shown, it is obvious to a person skilled in the art that the present invention is not limited to the described embodiment and may be changed and modified in various forms without departing from the spirit and scope of the present invention. Accordingly, modifications or variations should not be individually understood in view of the technical spirit of the present invention, and it must be understood the modifications and the variations belong to the claims of the present invention.

### Industrial Applicability

According to the present invention, a syringe needle assembly 100, and a safety syringe 10 and a safety syringe device 1 which use the syringe needle assembly 100 may be provided, in which the syringe needle assembly 100 includes a syringe needle 110 having a needle tip 111 formed on the front end thereof and a safety tube 130 covering the syringe needle 110, coupled to the rear end of the syringe needle 110 and opened in the injection direction of the syringe needle 110. When the syringe needle 110 is inserted into the skin B, the safety tube 130 is contracted towards the rear side of the needle tip 111, and when the syringe needle 110 is pulled out from the skin B, the safety tube 130 is extended towards the front side of the needle tip 111. Thus, the syringe needle assembly 100, the safety syringe 10, and the safety syringe device 1 of the present invention are simple in construction and may be safely and conveniently used while being applied to and used with an existing syringe by upgrading the existing syringe without changing the construction of the existing syringe. As a result, the present invention overcomes limitations of the existing techniques so that the present invention may be used in the related art and a device, to which the present invention is applied, may be adequate enough to be placed on the market or used in business. Furthermore, it is evident that the present invention can be practically carried out. Thus, the present invention is industrially applicable.

## Claims

1. A syringe needle assembly (100) comprising:
syringe needle (110) having a needle tip (111) formed on a front end thereof; and
a safety tube (130) covering the syringe needle (110), coupled to a rear end of the syringe needle (110) and opened in an injection direction of the syringe needle (110),
wherein, when the syringe needle (110) is inserted into skin, the safety tube (130) is contracted towards a rear side of the needle tip (111), and when the syringe needle (110) is pulled out from the skin, the safety tube (130) is extended towards a front side of the needle tip (111),
wherein the safety tube (130) comprises:
a deformation part (131) configured to be elastically deformed when the syringe needle (110) is inserted into the skin or pulled out from the skin; and
a fixing part (133) configured to support the deformation part (131) in the longitudinal direction of the syringe needle (110),
wherein the syringe needle assembly (100) further comprises:
a slider (150) formed to be movable along the outer circumferential surface of the safety tube (130), and in a case where the slider part is positioned on the outer circumferential surface of the deformation part (131), the inner circumferential surface of the slider (150) compresses the outer circumferential surface of the deformation part (131) when the deformation part (131) is elastically deformed,
**characterized in that** an inclined member (S) is formed on the front end portion of the safety tube (130), the inclined member (S) having an outer diameter which increases along the injection direction of the syringe needle (110) and an inner diameter which is reduced when the slider (150) is positioned on an outer circumference of the inclined member (S),
and wherein an engagement recess or an engagement hole (151) is formed on the slider (150), and
a protrusion (P) is formed on the front end portion of the safety tube (130) to be inserted and engaged in the engagement recess or the engagement hole (151) when the slider (150) is positioned on the outer circumferential surface of the inclined member (S).

2. The syringe needle assembly (100) of claim 1, wherein the safety tube (130) is formed of rubber, a resin or a silicon material.

3. The syringe needle assembly (100) of claim 1, wherein the deformation part (131) is formed of a soft flexible material, and
the fixing part (133) is formed of a material harder than the deformation part (131).

4. The syringe needle assembly (100) of claim 1, wherein the deformation part (131) is formed in a thickness thinner than the fixing part (133).

5. The syringe needle assembly (100) of claim 1, wherein a deformation guide recess (G) is formed along a circumferential direction on an inner circumferential surface or an outer circumferential surface of the deformation part (131) so that elastic deformation is intensively generated at the deformation guide recess (G).

6. The syringe needle assembly (100) of claim 1, wherein the deformation part (131) is formed on a front end portion of the safety tube (130), and
the front end portion of the deformation part (131) flares outward so that the inner circumferential surface is in contact with the skin.

7. The syringe needle assembly (100) of claim 6, wherein the deformation part (131) has an inner diameter which increases or is constant in the injection direction of the syringe needle (110).

8. The syringe needle assembly (100) of claim 1, wherein the deformation part (131) is formed on a middle portion of the safety tube (130) and has an inner diameter larger than the fixing part (133).

9. The syringe needle assembly (100) of claim 1, wherein the deformation part (131) is formed on a middle portion of the safety tube (130), and
a cutout is formed on an inner circumferential surface or an outer circumferential surface of the deformation part (131).

10. The syringe needle assembly (100) of claim 1, wherein at least one rib (E) is formed on the outer circumferential surface of the safety tube (130) to form a boundary for a moving distance of the slider (150) between the deformation part (131) and the fixing part (133).

11. The syringe needle assembly (100) of claim 1, wherein a rib (E) is formed on the inner circumferential surface, and
an insertion recess or an insertion hole (H) is formed on the outer circumferential surface of the safety tube (130) so that the rib (E) is inserted into the insertion recess or the insertion hole (H) to form a boundary for a moving distance of the slider (150) between the deformation part (131) and the fixing part (133) .

12. The syringe needle assembly (100) of claim 1, wherein the safety tube (130) comprises:
a first tube (T1); and
a second tube (T2) slid along a longitudinal direction of the first tube (T1).

13. The syringe needle assembly (100) of claim 12, wherein the first tube (T1) and the second tube (T2) are respectively formed with screw threads which are engaged with each other by relative rotation when the safety tube (130) is extended.

14. The syringe needle assembly (100) of claim 12, wherein one of the first tube (T1) and the second tube (T2) is formed with a protrusion and the other is formed with an insertion slot, and when the safety tube is extended, the protrusion is inserted and engaged in the insertion slot so that the extended state of the safety tube is maintained.

15. The syringe needle assembly (100) of claim 12, wherein one of the first tube (T1) and the second tube (T2) is formed with a sliding rib, and the other is formed with a sliding recess so that the sliding rib is inserted into the sliding recess to move along the sliding recess.

16. The syringe needle assembly (100) of claim 15, wherein the sliding recess comprises:
a moving section configured to allow sliding movement of the second tube (T2) along the longitudinal direction of the first tube (T1); and
a limit section configured to limit the sliding movement of the second tube (T2) along the longitudinal direction of the first tube (T1).

17. A safety syringe (10) comprising:
a syringe needle assembly (100) according to claim 1;
a syringe barrel (200), to which the syringe needle assembly (100) is coupled; and
a piston (300) configured to move within the syringe barrel (200).

18. A safety syringe device (1) comprising:
a syringe needle assembly (100) according to claim 11;
a syringe barrel (200), to which the syringe needle assembly (100) is coupled;
a piston (300) configured to move within the syringe barrel (200); and
a safety stand having an insertion hole (H) formed in a longitudinal direction, the insertion hole (H) having a size larger than the inner diameter of the slider (150) and smaller than the outer diameter of the slider (150),
wherein, when a lower end of the safety tube (130) is inserted into the insertion hole (H) in a state where the needle tip (111) is directed upward, the slider (150) is moved to the deformation part by the weight of the safety syringe (10).

## Patentansprüche

1. Spritzennadelanordnung (100), umfassend:
Spritzennadel (110), die eine Nadelspitze (111) aufweist, die an einem vorderen Ende davon gebildet ist; und
eine Sicherheitsröhre (130), die die Spritzennadel (110) bedeckt, an ein hinteres Ende der Spritzennadel (110) gekoppelt und in eine Injektionsrichtung der Spritzennadel (110) geöffnet ist,
wobei, wenn die Spritzennadel (110) in die Haut eingefügt wird, die Sicherheitsröhre (130) in Richtung einer Rückseite der Nadelspitze (111) eingezogen wird, und wenn die Spritzennadel (110) aus der Haut herausgezogen wird, die Sicherheitsröhre (130) in Richtung einer Vorderseite der Nadelspitze (111) erweitert wird,
wobei die Sicherheitsröhre (130) folgendes umfasst:
einen Verformungsteil (131), der konfiguriert ist, um elastisch verformt zu werden, wenn die Spritzennadel (110) in die Haut eingefügt oder aus der Haut herausgezogen wird; und
einen Halterungsteil (133), der konfiguriert ist, um den Verformungsteil (131) in die Längsrichtung der Spritzennadel (110) zu stützen,
wobei die Spritzennadelanordnung (100) ferner folgendes umfasst:
einen Schieber (150), der gebildet ist, sodass er bewegbar entlang der äußeren Umfangsfläche der Sicherheitsröhre (130) ist, und wobei in einem Fall, in dem der Schieberteil an der äußeren Umfangsfläche des Verformungsteils (131) positioniert ist, die innere Umfangsfläche des Schiebers (150) die äußere Umfangsfläche des Verformungsteils (131) komprimiert, wenn der Verformungsteil (131) elastisch verformt wird,
**dadurch gekennzeichnet, dass**
ein abgeschrägtes Element (S) an dem vorderen Endabschnitt der Sicherheitsröhre (130) gebildet ist, wobei das abgeschrägte Element (S) einen Außendurchmesser, der entlang der Injektionsrichtung der Spritzennadel (110) zunimmt, und einen Innendurchmesser, der reduziert ist, wenn der Schieber (150) an einem äußeren Umfang des abgeschrägten Elements (S) positioniert ist, aufweist,
und wobei eine Eingriffsaussparung oder ein Eingriffsloch (151) an dem Schieber (150) gebildet ist, und
ein Vorsprung (P) an dem vorderen Endabschnitt der Sicherheitsröhre (130) gebildet ist, um in die Eingriffsaussparung oder das Eingriffsloch (151) eingefügt und darin in Eingriff genommen zu werden, wenn der Schieber (150) an der äußeren Umfangsfläche des abgeschrägten Elements (S) positioniert ist.

2. Spritzennadelanordnung (100) nach Anspruch 1, wobei die Sicherheitsröhre (130) aus Gummi, einem Harz oder einem Silikonmaterial gebildet ist.

3. Spritzennadelanordnung (100) nach Anspruch 1, wobei der Verformungsteil (131) aus einem weichen flexiblen Material gebildet ist, und
der Halterungsteil (133) aus einem Material gebildet ist, das härter als der Verformungsteil (131) ist.

4. Spritzennadelanordnung (100) nach Anspruch 1, wobei der Verformungsteil (131) in einer Dicke gebildet ist, die dünner als der Halterungsteil (133) ist.

5. Spritzennadelanordnung (100) nach Anspruch 1, wobei eine Verformungsführungsaussparung (G) entlang einer Umfangsrichtung an einer inneren Umfangsfläche oder einer äußeren Umfangsfläche des Verformungsteils (131) gebildet ist, sodass intensiv elastische Verformung an der Verformungsführungsaussparung (G) erzeugt wird.

6. Spritzennadelanordnung (100) nach Anspruch 1, wobei der Verformungsteil (131) an einem vorderen Endabschnitt der Sicherheitsröhre (130) gebildet ist, und
sich der vordere Endabschnitt des Verformungsteils (131) nach außen aufweitet, sodass die innere Umfangsfläche in Kontakt mit der Haut ist.

7. Spritzennadelanordnung (100) nach Anspruch 6, wobei der Verformungsteil (131) einen Innendurchmesser aufweist, der in der Injektionsrichtung der Spritzennadel (110) zunimmt oder konstant ist.

8. Spritzennadelanordnung (100) nach Anspruch 1, wobei der Verformungsteil (131) an einem Mittelabschnitt der Sicherheitsröhre (130) gebildet ist und einen Innendurchmesser aufweist, der größer als der Halterungsteil (133) ist.

9. Spritzennadelanordnung (100) nach Anspruch 1, wobei der Verformungsteil (131) an einem Mittelabschnitt der Sicherheitsröhre (130) gebildet ist, und
ein Ausschnitt an einer inneren Umfangsfläche oder einer äußeren Umfangsfläche des Verformungsteils (131) gebildet ist.

10. Spritzennadelanordnung (100) nach Anspruch 1, wobei zumindest eine Rippe (E) an der äußeren Umfangsfläche der Sicherheitsröhre (130) gebildet ist, um eine Grenze für einen beweglichen Abstand des Schiebers (150) zwischen dem Verformungsteil (131) und dem Halterungsteil (133) zu bilden.

11. Spritzennadelanordnung (100) nach Anspruch 1, wobei eine Rippe (E) an der inneren Umfangsfläche gebildet ist, und
eine Einfügungsaussparung oder ein Einfügungsloch (H) an der äußeren Umfangsfläche der Sicherheitsröhre (130) gebildet ist, sodass die Rippe (E) in die Einfügungsaussparung oder das Einfügungsloch (H) eingefügt wird, um eine Grenze für einen beweglichen Abstand des Schiebers (150) zwischen dem Verformungsteil (131) und dem Halterungsteil (133) zu bilden.

12. Spritzennadelanordnung (100) nach Anspruch 1, wobei die Sicherheitsröhre (130) folgendes umfasst:
eine erste Röhre (T1); und
eine zweite Röhre (T2), die entlang einer Längsrichtung der ersten Röhre (T1) geschoben wird.

13. Spritzennadelanordnung (100) nach Anspruch 12, wobei die erste Röhre (T1) und die zweite Röhre (T2) jeweils mit Schraubengewinden gebildet sind, die durch relative Rotation gegenseitig in Eingriff genommen sind, wenn die Sicherheitsröhre (130) erweitert ist.

14. Spritzennadelanordnung (100) nach Anspruch 12, wobei eine von der ersten Röhre (T1) und der zweiten Röhre (T2) mit einem Vorsprung gebildet ist und die andere mit einem Einfügungsschlitz gebildet ist, und wobei, wenn die Sicherheitsröhre erweitert ist, der Vorsprung in den Einfügungsschlitz eingefügt und darin in Eingriff genommen ist, sodass der erweiterte Zustand der Sicherheitsröhre beibehalten wird.

15. Spritzennadelanordnung (100) nach Anspruch 12, wobei eine von der ersten Röhre (T1) und der zweiten Röhre (T2) mit einer Schieberippe gebildet ist und die andere mit einer Schiebeaussparung gebildet ist, sodass die Schieberippe in die Schiebeaussparung eingefügt ist, um sich entlang der Schiebeaussparung zu bewegen.

16. Spritzennadelanordnung (100) nach Anspruch 15, wobei die Schiebeaussparung folgendes umfasst:
einen Bewegungsabschnitt, der konfiguriert ist, um Schiebebewegung der zweiten Röhre (T2) entlang der Längsrichtung der ersten Röhre (T1) zu ermöglichen; und
einen Begrenzungsabschnitt, der konfiguriert ist, um die Schiebebewegung der zweiten Röhre (T2) entlang der Längsrichtung der ersten Röhre (T1) zu begrenzen.

17. Sicherheitsspritze (10), umfassend:
eine Sicherheitsspritzenanordnung (100) nach Anspruch 1;
einen Spritzenlauf (200), an den die Spritzennadelanordnung (100) gekoppelt ist; und
einen Kolben (300), der konfiguriert ist, um sich innerhalb des Spritzenlaufs (200) zu bewegen.

18. Sicherheitsspritzenvorrichtung (1), umfassend:
eine Sicherheitsspritzenanordnung (100) nach Anspruch 11;
einen Spritzenlauf (200), an den die Spritzennadelanordnung (100) gekoppelt ist;
einen Kolben (300), der konfiguriert ist, um sich innerhalb des Spritzenlaufs (200) zu bewegen; und
einen Sicherheitsständer, der ein Einfügungsloch (H) aufweist, das in eine Längsrichtung gebildet ist, wobei das Einfügungsloch (H) eine Größe aufweist, die größer als der Innendurchmesser des Schiebers (150) und kleiner als der Außendurchmesser des Schiebers (150) ist,
wobei, wenn ein unteres Ende der Sicherheitsröhre (130) in das Einfügungsloch (H) in einem Zustand eingefügt wird, in dem die Nadelspitze (111) nach oben gerichtet ist, der Schieber (150) durch das Gewicht der Sicherheitsspritze (10) zu dem Verformungsteil bewegt wird.

## Revendications

1. Ensemble (100) avec aiguille et seringue comprenant :
une aiguille de seringue (110) dont une pointe (111) de l'aiguille est formée sur une extrémité avant de ladite aiguille ; et
un tube de sécurité (130) recouvrant l'aiguille (110) de la seringue, ledit tube de sécurité étant couplé à une extrémité arrière de l'aiguille (110) de la seringue et ouvert dans une direction d'injection de l'aiguille (110) de la seringue,
ensemble dans lequel, quand l'aiguille (110) de la seringue est introduite dans la peau, le tube de sécurité (130) est contracté vers un côté arrière de la pointe (111) de l'aiguille, et quand l'aiguille (110) de la seringue est retirée de la peau, le tube de sécurité (130) est déployé vers un côté avant de la pointe (111) de l'aiguille,
ensemble dans lequel le tube de sécurité (130) comprend :
une partie à déformation (131) configurée pour être élastiquement déformée quand l'aiguille (110) de la seringue est introduite dans la peau ou retirée de la peau; et
une partie de fixation (133) configurée pour supporter la partie à déformation (131) dans la direction longitudinale de l'aiguille (110) de la seringue,
où l'ensemble (100) avec aiguille et seringue comprend en outre :
un élément coulissant (150) formé pour être mobile le long de la surface circonférentielle extérieure du tube de sécurité (130) et, au cas où la partie de l'élément coulissant est positionnée sur la surface circonférentielle extérieure de la partie à déformation (131), la surface circonférentielle intérieure de l'élément coulissant (150) comprime la surface circonférentielle extérieure de la partie à déformation (131) quand la partie à déformation (131) est élastiquement déformée,
**caractérisé**
**en ce qu'**un élément incliné (S) est formé sur la partie d'extrémité avant du tube de sécurité (130), l'élément incliné (S) ayant un diamètre extérieur qui augmente le long de la direction d'injection de l'aiguille (110) de la seringue et ayant un diamètre intérieur qui est réduit quand l'élément coulissant (150) est positionné sur une circonférence extérieure de l'élément incliné (S),
et où une cavité d'engagement ou un trou d'engagement (151) est formé(e) sur l'élément coulissant (150), et
une partie saillante (P) est formée sur la partie d'extrémité avant du tube de sécurité (130) devant être introduit et engagé dans la cavité d'engagement ou dans le trou d'engagement (151) quand l'élément coulissant (150) est positionné sur la surface circonférentielle extérieure de l'élément incliné (S).

2. Ensemble (100) avec aiguille et seringue selon la revendication 1, dans lequel le tube de sécurité (130) est formé en étant composé d'un matériau à base de caoutchouc, de résine ou de silicone.

3. Ensemble (100) avec aiguille et seringue selon la revendication 1, dans lequel la partie à déformation (131) est formée en étant composée d'un matériau flexible et mou, et
la partie de fixation (133) est formée en étant composée d'un matériau plus dur que celui de la partie à déformation (131).

4. Ensemble (100) avec aiguille et seringue selon la revendication 1, dans lequel la partie à déformation (131) est formée en ayant une épaisseur plus mince que celle de la partie de fixation (133).

5. Ensemble (100) avec aiguille et seringue selon la revendication 1, dans lequel une cavité (G) de guidage de la déformation est formée le long d'une direction circonférentielle sur une surface circonférentielle intérieure ou sur une surface circonférentielle extérieure de la partie à déformation (131), de sorte qu'une déformation élastique est fortement générée au niveau de la cavité (G) de guidage de la déformation.

6. Ensemble (100) avec aiguille et seringue selon la revendication 1, dans lequel la partie à déformation (131) est formée sur une partie d'extrémité avant du tube de sécurité (130), et
la partie d'extrémité avant de la partie à déformation (131) s'évase vers l'extérieur, de sorte que la surface circonférentielle intérieure est en contact avec la peau.

7. Ensemble (100) avec aiguille et seringue selon la revendication 6, dans lequel la partie à déformation (131) a un diamètre intérieur qui augmente ou qui est constant dans la direction d'injection de l'aiguille (110) de la seringue.

8. Ensemble (100) avec aiguille et seringue selon la revendication 1, dans lequel la partie à déformation (131) est formée sur une partie centrale du tube de sécurité (130) et a un diamètre intérieur plus grand que celui de la partie de fixation (133).

9. Ensemble (100) avec aiguille et seringue selon la revendication 1, dans lequel la partie à déformation (131) est formée sur une partie centrale du tube de sécurité (130), et
une découpe est formée sur une surface circonférentielle intérieure ou sur une surface circonférentielle extérieure de la partie à déformation (131).

10. Ensemble (100) avec aiguille et seringue selon la revendication 1, dans lequel au moins une nervure (E) est formée sur la surface circonférentielle extérieure du tube de sécurité (130), pour former une limite à une distance de déplacement de l'élément coulissant (150), comprise entre la partie à déformation (131) et la partie de fixation (133).

11. Ensemble (100) avec aiguille et seringue selon la revendication 1, dans lequel une nervure (E) est formée sur la surface circonférentielle intérieure, et
une cavité d'insertion ou un trou d'insertion (H) est formé(e) sur la surface circonférentielle extérieure du tube de sécurité (130), de sorte que la nervure (E) est insérée dans la cavité d'insertion ou dans le trou d'insertion (H), pour former une limite à une distance de déplacement de l'élément coulissant (150), comprise entre la partie à déformation (131) et la partie de fixation (133).

12. Ensemble (100) avec aiguille et seringue selon la revendication 1, dans lequel le tube de sécurité (130) comprend :
un premier tube (T1) ; et
un second tube (T2) coulissant le long d'une direction longitudinale du premier tube (T1).

13. Ensemble (100) avec aiguille et seringue selon la revendication 12, dans lequel le premier tube (T1) et le second tube (T2) sont respectivement formés en ayant des filets de vis qui sont engagés les uns dans les autres par rotation relative, quand le tube de sécurité (130) est déployé.

14. Ensemble (100) avec aiguille et seringue selon la revendication 12, dans lequel l'un ou l'autre du premier tube (T1) et du second tube (T2) est formé en ayant une partie saillante, l'autre tube étant formé en ayant une fente d'insertion, et quand le tube de sécurité est déployé, la partie saillante est insérée et engagée dans la fente d'insertion, de sorte que l'état déployé du tube de sécurité est maintenu.

15. Ensemble (100) avec aiguille et seringue selon la revendication 12, dans lequel l'un ou l'autre du premier tube (T1) et du second tube (T2) est formé en ayant une nervure de coulissement, l'autre tube étant formé en ayant une cavité de coulissement, de sorte que la nervure de coulissement est insérée dans la cavité de coulissement pour se déplacer le long de la cavité de coulissement.

16. Ensemble (100) avec aiguille et seringue selon la revendication 15, dans lequel la cavité de coulissement comprend :
une section de déplacement configurée pour permettre un mouvement coulissant du second tube (T2) le long de la direction longitudinale du premier tube (T1) ; et
une section formant une limite configurée pour limiter le mouvement coulissant du second tube (T2) le long de la direction longitudinale du premier tube (T1).

17. Seringue de sécurité (10) comprenant :
un ensemble (100) avec aiguille et seringue selon la revendication 1 ;
un cylindre de seringue (200) auquel est couplé l'ensemble (100) avec aiguille et seringue ; et
un piston (300) configuré pour se déplacer à l'intérieur du cylindre (200) de la seringue.

18. Dispositif de seringue de sécurité (1) comprenant :
un ensemble (100) avec aiguille et seringue selon la revendication 11 ;
un cylindre de seringue (200) auquel est couplé l'ensemble (100) avec aiguille et seringue ;
un piston (300) configuré pour se déplacer à l'intérieur du cylindre (200) de la seringue ; et
un support de sécurité ayant un trou d'insertion (H) formé dans une direction longitudinale, le trou d'insertion (H) ayant une dimension plus grande que le diamètre intérieur de l'élément coulissant (150) et plus petite que le diamètre extérieur de l'élément coulissant (150),
dispositif dans lequel, quand une extrémité inférieure du tube de sécurité (130) est introduite dans le trou d'insertion (H), dans un état où la pointe (111) de l'aiguille est dirigée vers le haut, l'élément coulissant (150) est déplacé, par le poids de la seringue de sécurité (10), jusqu'à la partie à déformation.
